# EUROPEAN PATENT APPLICATION

(11) **EP 4 086 913 A1**
(43) Date of publication of application: **09.11.2022**
(21) Application number: 22171601.2
(22) Date of filing: 04.05.2022
(51) Int. Cl.: G16H 20/17

(54) **PERSONALIZED CONTEXT SENSITIVE MEAL TRACKING FOR AUTOMATIC DRUG DELIVERY SYSTEMS**

(30) Priority: 05.05.2021 US 202163184241 P
(71) Applicant: Insulet Corporation, Acton MA 01720 (US)
(72) Inventor: NARAYANASWAMI, Rangarajan, Weston (US); JANTZ, Jay, Acton (US); LOWEN, Steven, Bedford (US); CHANG, Albert, Billerica (US); KARKACH, Oussama, Salem (US)
(74) Representative: Peterreins Schley

(57) **Abstract**

The disclosed embodiments are directed to an automatic drug delivery (ADD) system device configured to provide bolus dosing of insulin. The embodiments include a system and method for providing an improved meal input interface for the user as well as methods for the use of the information provided by the user to both improve the post-prandial bolus dosing of insulin and to advise the user on meals that will lead to improved blood glucose control for the user.

## Description

### BACKGROUND

Insulin delivery for meal compensation is a critical component in blood glucose control in automatic drug delivery (ADD) systems for people with diabetes. The bolus insulin delivered when a meal is ingested is typically determined by the number of grams of carbohydrate in the meal and the user's insulin to carbohydrate ratio. Particularly for open loop systems, the accurate entry of the number of carbohydrates in a meal is important to keep the blood glucose levels in an acceptable range. When the insulin dosage is matched appropriately with the meal, a matching flow of insulin is provided when the glucose from the meal absorption enters the bloodstream. However, closed loop systems will also benefit from accurate estimation of the number of carbohydrates for optimal control of the blood glucose level.

It is well-known that factors other than the number of grams of carbohydrate in a meal affect determination of the size of the bolus dose and the timing of the delivery of the bolus dose of insulin required to offset the post-prandial absorption of glucose into the bloodstream. For example, the overall macronutrient profile of a meal, including the ratios of fat and protein to carbohydrates, and the quantities of fat, protein and carbohydrates in the meal will affect not only the amount of glucose which enters the bloodstream but also the rate and timing of absorption of glucose from the meal into the bloodstream. Ideally, a bolus dose of insulin would be delivered to the user that matches the timing of absorption of the glucose into the bloodstream.

A typical user interface for entry of meal information to an automatic drug delivery (ADD) system only requires entry of the number of grams of carbohydrates contained in the meal. This method is error-prone for the user and is not nutritively informative. The lack of macronutritional information about the meal precludes presenting the user with a holistic report of ingested meals with concomitant blood glucose profiles and does not enable the calculation of an effective bolus split or the timing of the delivery of the insulin to the user.

Therefore, in addition to having the user provide the number of grams of carbohydrates for each meal, it would be beneficial for the user to have the ability to enter meal descriptors, which describe the overall macronutrient profile of the meal and which could then be entered into a catalog of ingested meals. The catalog of ingested meals can further be analyzed for balanced nutrition, calorie intake, food tolerance, effect on the blood glucose levels, etc. Additionally, the meal descriptors could be used as input to determine the bolus split parameters required to match the absorption of glucose into the user's bloodstream.

In addition, an ADD system may include a drug delivery device, including wearable drug delivery devices, which provide delivery of both basal and bolus doses of insulin. It would be desirable to have the macronutrient profile of the meal collected from the user to be provided to the drug delivery device such as to enable the drug delivery device to automatically adjust both the bolus split for each meal.

### DEFINITIONS

As used herein, the term *"**insulin**"* should be interpreted to include insulin, or co-formulations of GLP-1 and insulin, or co-formulations of pramlintide and insulin.

As used herein, the term *"**bolus split**"* refers to splitting a bolus dose of insulin into a first portion, to be delivered immediately, and one or more additional portions, to be delivered at a later time or over an extended period of time starting at some point after the first portion is delivered.

### SUMMARY

This Summary is provided to introduce a selection of concepts in a simplified form that are further described below in the Detailed Description. This Summary is not intended to identify key features or essential features of the claimed subject matter, nor is it intended as an ADD in determining the scope of the claimed subject matter.

Disclosed herein is a method for providing an improved meal input interface for the user as well as methods for the use of the information provided by the user to both improve the post-prandial bolus dosing of insulin and to advise the user on meals that will lead to improved blood glucose control for the user.

In one aspect of the invention, the method provides a personalization filter that allows the user to enter dietary preferences, for example, vegetarian, favorite cuisine choices, allergies, etc. These dietary preferences may be used in accordance with other parameters to select foods or combinations of foods for suggestion to the user.

In another aspect of the invention, the method provides a suggestion filter that provides intelligent suggestions of likely food items or overall meals, considering user dietary preferences, history of ingested meals, time of day, location, day of the week, special days, etc.

In another aspect of the invention, the method provides a complementary association filter that develops, over time, an association of foods that go together or are complementary with respect to each other (e.g. soup & sandwich, burger & fries, bagel & cream cheese, etc.) These complementary items can easily be added by the user.

In another aspect of the invention, the method provides a meal selection interface that presents an intuitive interface with starting keyword-based selection, scrolling graphical views of foods and contextual hints that allow a user to easily select food items for inclusion in a meal. The meal selection interface may be adaptively driven by the personalization filter, the suggestion filter, and the complementary association filter to provide food items likely to be chosen by or preferred by the user for inclusion in a meal.

In another aspect of the invention, the method provides a portion estimator assistance tool that allows the user to intelligently specify a size of the portion of each food item or the number of pieces of the food item in the meal.

In another aspect of the invention, the method provides a nutrient extraction facility that can create a macronutrient profile of a meal including, for example, number of grams of carbs/protein/fat in the meal, based on the food items selected and the portions of each individual food item. The information regarding the macronutrient contents of individual food items may be obtained by integration with third party applications.

In another aspect of the invention, the method provides a smart notes logger which allows the user to create notes at the time of recording of a meal, with descriptors for later review, storage and retrieval.

In another aspect of the invention, the method provides a meal catalog which logs meals entered by the user to a catalog of meals that the user has ingested. This will serve as a valuable resource to correlate blood glucose control with meal type, and for the review of insulin delivery for meal compensation.

In a final aspect of the invention, the method may provide a feedback facility that detects instances where the macronutritional content of the meal has been mis-estimated, as ascertained by an excursion in the blood glucose curve and provides feedback to the user to improve their future estimates.

A primary embodiment of the invention may comprise any combination of the aspects discussed above, while other embodiments of the invention may contain subsets of the various aspects.

### BRIEF DESCRIPTION OF THE DRAWINGS

To easily identify the discussion of any particular element or act, the most significant digit or digits in a reference number refer to the figure number in which that element is first introduced.
**FIG. 1** is a block diagram of an ADD system including the macronutrient management application of the present invention.
**FIG. 2** is a block diagram showing the software architecture of the macronutrient management application of the present invention.
**FIG. 3** is an exemplary screen showing the entry of user preferences.
**FIG. 4A** is an exemplary screen from the meal selection interface, showing graphical representations of suggested food items which may be selected by the user for inclusion in the overall meal.
**FIG. 4B** is an exemplary screen for the meal selection interface showing the suggestion of complementary food item as suggested by the complementary association filter.
**FIG. 5A** is an exemplary screen from the meal selection interface showing food item selections based on location and time-of-day.
**FIG. 5B** is an exemplary screen from the meal selection interface showing input from the portion selection tool, showing an intelligent selection of possible portion sizes from which the user can select.
**FIG. 6** is an exemplary screen from the nutrient extraction facility, showing the overall macronutrient profile of the meal selection from **FIGS. 5A-5B****.**

### DETAILED DESCRIPTION

Devices and methods in accordance with the present disclosure will now be described more fully with reference to the accompanying drawings, where one or more embodiments or various aspects of the invention are shown. The systems and methods may be embodied in many different forms and are not to be construed as being limited to the embodiments set forth herein. Instead, these embodiments are provided so the disclosure will be thorough and complete, and will fully convey the scope of the systems and methods to those skilled in the art. Each of the systems and methods disclosed herein provides one or more advantages over conventional systems and methods.

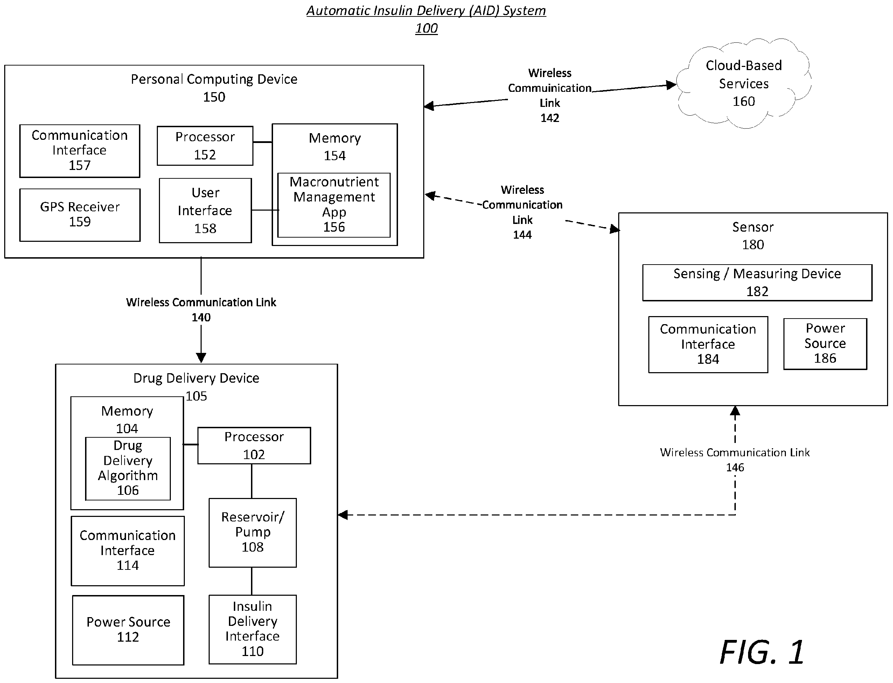

**FIG. 1** is a block diagram showing an ADD system which includes a macronutrient management application 156 implementing the novel methods of the invention described in the Summary above and in more detail below. In a primary embodiment of the invention, the macronutrient management application 156 may be implemented as a software application executing on a personal computing device 150. Personal computing device 150 may be configured with a processor 152 and a memory 154 containing the macronutrient management application 156. Personal computing device 150 may be further configured with a user interface 158 which may be used by the macronutrient management application 156 to enable interaction with a user. The macronutrient management application 156 may be configured to accept meal information input via user interface 158. The macronutrient management application 156 may optionally be further configured to receive post-prandial blood glucose traces of the user. In some embodiments, the blood glucose traces may be received from a sensor 180, for example, a continuous blood glucose monitor (CGM), directly via communication link 144 with sensor 180, or indirectly via drug delivery device 105 via wireless communication link 140, which communicates with sensor 180 via wireless connection 146. Alternatively, the user may enter the blood glucose levels directly via user interface 158. The macronutrient management application 156 may further communicate with cloud-based services 160 via communication link 142 as described below.

In some embodiments, personal computing device 150 may comprise, for example, a smartphone, a tablet device, a smartwatch, or any other personal mobile computing device capable of running macronutrient management application 156 and communicating with the drug delivery device 105, cloud-based services 160, and sensor 180 via any well-known wireless communication protocol.

The macronutrient management application 156 may track and correlate foods eaten by the user with blood glucose levels of the user for a predetermined period of time after the user has ingested the meal, and may inform a drug delivery algorithm 106 executing on drug delivery device 105 such as to enable the drug delivery algorithm 106 to adjust the post-prandial bolus dose, delivered as a bolus split of insulin delivered to the user to more effectively control the blood glucose levels of the user.

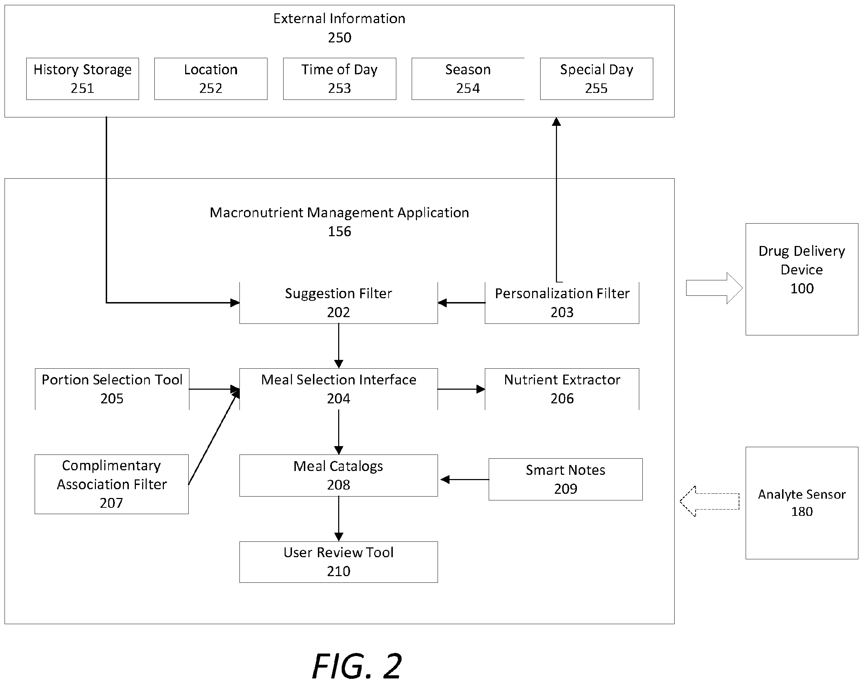

**FIG. 2** is a block diagram showing the overall architecture of the macronutrient management application 156. The application draws upon personal preferences, a suggestion filter that tailors food choice selections to the user, and a complementary association filter that identifies complementary foods for easy selection. The meal selection interface 204 can search for meals by starting letters, direct meal entry, special cuisine, restaurant menu, etc. The suggestion filter 202 will intelligently filter choices presented to the user or prepopulate likely choices for easy selection. Nutrient information used by the nutrient extractor component 206 may be supplied locally or by integration with third party applications. A smart portion selection tool 205 will help the user to correctly specify the portion size or number of pieces of the food item.

Personalization filter 203 allows the user to enter information regarding personal preferences for choices of food. Representative preference categories that may be set by the user include, but are not limited to, diet style (e.g. vegetarian, vegan, Mediterranean, kosher, etc.), favorite cuisines (e.g. Chinese, Mexican, Italian, Indian, etc.), allergies (e.g. dairy, eggs, nuts etc.), preferred food choices, specific food dislikes, etc. The personalization filter 203 is used by the suggestion filter 202 to filter the selection of food items offered to the user to the food items that the user is most likely to be interested in eating or to a choice of food items that are appealing to and appropriate for the user. As specific examples, a vegetarian preference will filter out meal choices that include meat, while an indication of an egg allergy might filter out egg sandwiches and breakfast meals that include eggs, etc.

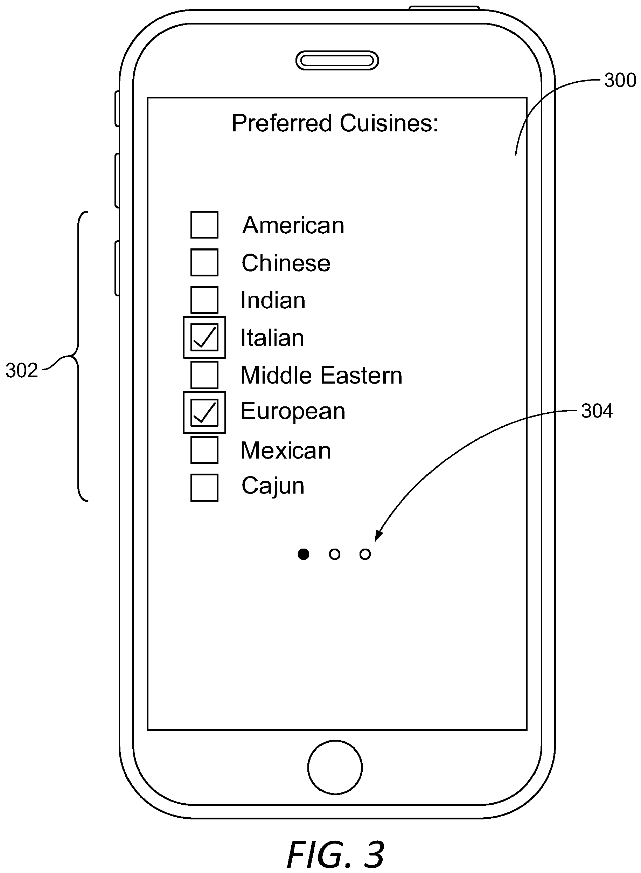

On initial use of the macronutrient management application 156, the user may be provided with a set of questions and may answer the questions to personalize the application to choices relevant to the user. Thereafter, if the user wishes to change their personal preferences, the macronutrient management application 156 may provide the user with a screen 300, an exemplary embodiment of which is shown in **FIG. 3****,** where the user may be provided with a method for specifying preferences, for example, check boxes 302, allowing the user to check or uncheck various preferences. **FIG. 3** shows an exemplary user interface screen showing a specific category of "Preferred Cuisines"; however, as indicated by dots 304, the user may swipe left or right to move to different categories of preferences, for example, allergies or preferred food choices. In addition, macronutrient management application 156 may provide the user with an opportunity to provide a positive or negative preference for specific food items when those food items are presented to the user as suggestions. User preferences may be stored in history storage 251.

Suggestion filter 202 intuitively suggests food items or overall meals that the user may consider eating. The suggestion filter 202 takes into account external information 250 to customize the list of food items that are presented to the user for consideration. Consequently, the user choices are tailored to and, thus, more relevant to the user. This makes it easy for the user to select the meal that he/she is planning to eat or has already eaten.

The suggestion filter 202 takes into account the user preferences and other history information stored in history storage 251. In addition, suggestion filter 202 may also take into account the location 252 of the user (e.g. home/work/restaurant/travel), which may be provided by, for example, GPS receiver 159 in personal computing device 150. Other information taken into account by suggestion filter 202 may include the time of day 253, the season of the year 254 and whether or not the current day is a special day 255. Suggestion filter 202 may access some information from other utilities or applications on the personal computing device 150 on which the macronutrient management application 156 is being executed, for example, the clock and/or the user's calendar application. Criteria for use by the suggestion filter 202 is not intended to be limited to the examples discussed herein but may include any criteria as an input for use in selecting suggested food items.

As an example, if it is 7 am, presumably the meal is breakfast. The past breakfast history of the user therefore may become an initial candidate list of possible food items for selection by the user. If GPS receiver 159 of personal computing device 150 provides a location that is "home", then the meal choices may be further narrowed to past at-home breakfast meals. As an alternate example, if the location provided is near a restaurant, suggestion filter 202 could tailor the meal choices to the restaurant menu and may make selections from the restaurant menu which are customized to the user with regard to past history, user preferences, etc.

The suggestion filter 202 may provide selections of food items based on a machine learning model which may be trained to provide food item suggestions based on various criteria, including, but not limited, to the user's history information 251 (including, for example, user preferences and past meals eaten), location 252, time of day 253, the season 254, and special day indications 255. The machine learning model may correlate the criteria (location, time of day, season, etc.) with past meals eaten to provide suggestions of one of more food items and portion sizes.

The suggestion filter 202 feeds one or more suggested food items into meal selection interface 204, which allows the user to select food items comprising a meal by selecting graphical representations of the food items. For example, selectable thumbnail images of the food items may be displayed. An initial set of food items may be provided by selection filter 202. Additionally, the user may guide the selection filter 202 by pressing a start letter for a meal.

Suggestion filter 202 provides a tailored set of food items, presented graphically to the user, from which the user may select one or more of the food items for inclusion in the meal. If the user is planning on a new meal for this occasion or does not wish to select any of the presented food items, other food items may be searched for using a full or partial text search.

The complementary association filter 207 finds groupings of food items that are complementary with respect to each other (i.e., food items that "go together"). For example, bagel and cream cheese, soup and sandwich, burger and fries, etc. are food items that are commonly eaten paired with each other and, as such, are "complementary". The complementary association filter 207 feeds into the meal selection interface 204 such that, when a user enters or selects a particular food item, the meal selection interface 204 may automatically present other food items as choices to the user that are complementary to the selected food item. The determination of whether food items go together, or which food items go with which other food items may be made based on an analysis of selections made by a wide population of users by determining which food items have been selected with other food items by the overall population of users. The meal selections of users may be uploaded to cloud-based service 160 via wireless communication link 142 and used to populate a database and/or to train a machine learning model to output the suggestion of one or more food items that go with a selected input food item.

The meal selection interface 204 is a graphical interface that allows the user to enter the food items that the user intends to eat or already has eaten. Using the meal selection interface 204, the user can select multiple food items with the touch of a button and a few clicks. Operation of the meal selection interface 204 is described with respect to the examples below.

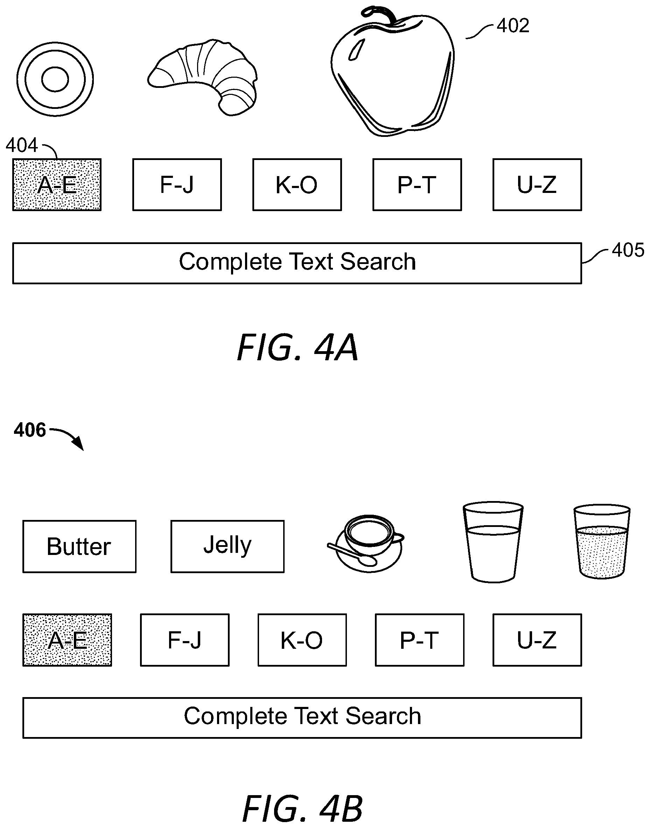

One exemplary embodiment of a meal selection interface is shown in FIG. 4A. In the case shown, for example, suggestion filter 202 recognizes it is 7 am, implying that the user will be eating breakfast. The user may trigger a search by hitting the "A-E" button 404, indicating that the user is interested in foods beginning with "A", "B", "C", "D" or "E". The suggestion filter 202 utilizes the past history 251 for home location 252 and starting letter for text search 404 and produces one or more suggested food items 402, in this case, a bagel, a croissant and an apple, which are graphically represented. Had the user made a different starting letter selection 404, for example, "U-Z", suggestion filter 202 may have suggested, for example, waffles. Should the user not wish to select either the bagel, croissant or apple, the user may select another set of initial letters 404, or may begin a complete or partial text search by selecting button 405, which may cause a keyboard to pop up on the user interface 158 of personal computing device 150 to allow the user to enter the search string. Graphical representations of the food items matching with the search string are displayed and may be selected by the user.

In one embodiment of the invention, a running histogram of a user's favorite meals may be maintained and filtered by location, time of day, on-boarding personalization filter, etc., to provide the user with appropriate suggestions from which a selection may be made.

In this exemplary embodiment, if the user selects "bagel" as the choice, the complementary association filter 207 may provide suggestion filter 202 with one or more complementary food items 406, from which the user may choose, as shown in **FIG. 4B****.** In this case, the complementary food items are butter and jelly as add-on food items, and coffee, juice, and milk as add-on beverages. Note that many people are likely to have selected cream cheese as a complementary food item for a bagel; however, in this case, the user may have expressed a negative user preference for cream cheese, or may have an allergy issue, and thus, suggestion filter 202 may have filtered out the cream cheese food item.

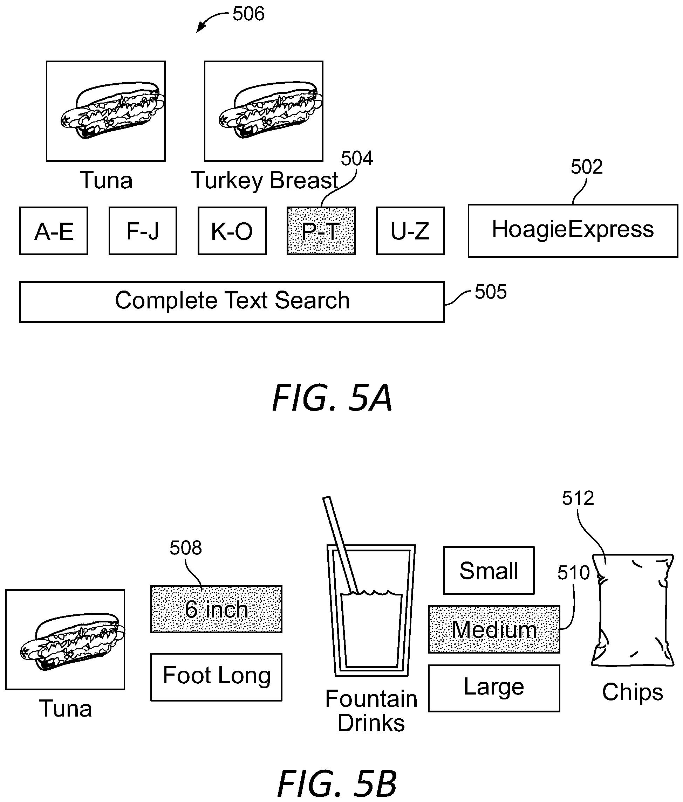

Another exemplary embodiment of the meal selection interface 204 is shown in FIG. 5A. If the time is near noon and the provided location is near a particular restaurant, in this example, "HoagieExpress", the suggestion filter 202 may cause the meal selection interface 204 to display the logo 502 for the particular restaurant, as well as graphical representations of various food items from the restaurant's menu. Macronutrient management application 156 may interface with third party databases or applications to obtain the food items on the restaurant's menu. The food items displayed may be chosen by suggestion filter 202 based on, for example, location 252, time of day 253 and the user's past history 251. For example, the machine learning model utilized by suggestion filter 202 may correlate the particular time of day and location with the user's past history of ordering from the restaurant. Initially, meal selection filter 204 may present food items previously selected by the user. The user may select one of the presented food items or may search for alternate food items. If the user thereafter selects, for example, initial letters "P-T" 504, the suggestion filter may provide selections of tuna and turkey sandwiches 506, based on the past history 251 of the user ordering these particular sandwiches from the particular restaurant. In this example, the user is presented with a selection of a tuna or turkey sandwich 506. Should the user not wish to select either the tuna or turkey sandwich, the user may select another set of initial letters 504, or may begin a complete or partial text search by selecting button 505, which may cause a keyboard to pop up on user interface 158 of personal computing device 150 to allow the user to enter the search string.

After the user has selected a tuna sandwich from the interface shown in **FIG. 5A****,** the user interface shown in **FIG. 5B** is presented. This interface shows suggestions made by the portion selection tool 205, to allow the user to select a 6-inch or "foot long" tuna sandwich. Complementary association filter 207, may select complementary food items, for example a beverage or chips, based on the user's selection of a tuna sandwich. Portion selection tool 205 may also suggest various sizes for the beverage. As such, in addition to selection of the sandwich, the user can select a beverage (and a size for the beverage) using button 510 and/or potato chips 512. Certain buttons may be highlighted based on, for example, the user's history 251, which may contain knowledge of complementary food items previously ordered and the sizes previously ordered when the user has ordered a tuna sandwich at the restaurant.

In yet other embodiments, meal selection interface 204 may include smart embodiments to directly link to carry out or delivery restaurant menus, prepared store dinners, etc. In cases where the user is exploring a new meal for the first time (i.e. there is no history information regarding this meal in user history 251), then a full or partial text search may be entered by the user, as specified in the examples above, to specify one or more food items. These food items then become part of the user history 251 for subsequent selections of food items.

The portion selection tool 205 smartly offers size choices or the choice of the number of pieces so the user can finalize the choice of food items and portion size. As an example, when the bagel is chosen in the example above, the portion estimator tool 205 will offer the number of bagels as a choice. For a beverage, the portion size will switch to small, medium, and large. For the restaurant sandwich example above, it will contextually switch to 6-inch or foot long sandwiches, as shown in **FIG. 5B****.** Choices of other food items may naturally suggest different portion sizes. For example, pizza may be naturally divided into a number of slices. For home-served portions such as cereal, oatmeal, pasta, rice, vegetables etc. it may be difficult to estimate the portion size. For this scenario, portion selection tool 205 may display standard plates/cups/bowls with graphical representations of small, medium, and large portions from which the user can select to determine a matching portion size.

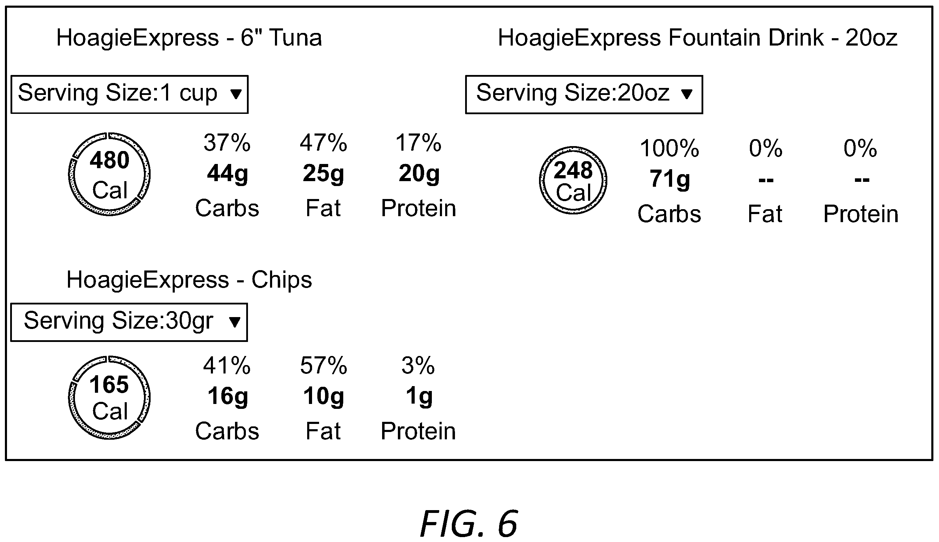

Once the meal (including one or more food items and beverages) and portion sizes are finalized, the nutrient extractor 206 component of the macronutrient management application 156 creates a macronutrient profile for the meal, which preferably includes, but is not limited to, estimates of the quantity of carbohydrates, proteins and fats contained in the food items selected for the meal. Information regarding the macronutrient composition of individual food items may be extracted from a local database or may be obtained from third party nutrition applications. As an example, the restaurant's six-inch tuna sandwich + medium beverage + bag of chips from the example above will return the macronutrient information shown in **FIG. 6****.** The total number of carbohydrates is (44g+71g+16g) = 131g, the total number of fat grams is (25g+10g) = 35g, and the total number of protein grams is 21g. This information is automatically extracted once the food items for the meal are selected and the portion sizes determined. As a step to automatically interfacing to a third-party application, the meal selection interface 204 may compose a query that infers the correct name for the food items in the meal or the overall meal and the correct portion size. Nutrient components of each food item in the meal will all be added together to infer the macronutrient profile of the meal, including the total number of grams of carbohydrate/fat/protein in the meal.

The macronutrient profile of the meal may be sent to drug delivery device 105 and may be used by the drug delivery algorithm 106 on drug delivery device 105 to determine an accurate bolus dose, as well as the timing for the delivery of the bolus split, given the selected meal. In alternative embodiments, macronutrient management application 156 may directly determine the bolus dose and bolus split, given the selected meal, and may send that information to drug delivery device 105. Macronutrient management application 156 communicates the information regarding the macronutrient profile of the meal or the determined bolus dose and bolus split via communication interface 157 on personal computing device 152, which communicates with communication interface 114 on drug delivery device 105 via wireless communication link 140.

In one embodiment, macronutrient management application 156 may be provided with a smart notes logger 209 which the user can use to can tag smart notes to the meal choices for their own review later or for annotating specific interesting pieces of information. For example, "did not complete full meal", "added extra condiments", "portion estimation is not correct", etc. The smart notes logger 209 may also be used, for example, to tag various food items as favorite or as unfavorite food items, to describe any allergic reaction to a food item, etc. This information may be stored in history storage 251 and maybe used as input to suggestion filter 202 when suggesting food items for meals.

Each meal consumed by the user will be logged to a meal catalog 208. The meal catalog 208 will contain complete meal descriptions, macronutrient profiles, descriptions, total calorie intake and any user notes. Statistics from the meal catalog 208 may include meals per day, timing of meals, missed meals, total carbs/fat/protein/ per day, total calories per day, etc. The meal catalog 208, along with an insulin delivery schedule and blood glucose traces, can be illuminating to the user and/or the user's healthcare professional in maintaining effective control of the user's blood glucose levels. The meal catalog 208 also serves as a source for querying history for use by the suggestion filter 202.

In certain embodiments, macronutrient management application 156 may receive information regarding the post-prandial blood glucose trace of the user from sensor 180 which may be, for example, a continuous glucose monitor (CGM) which is worn as a wearable device by the user. Sensor 180 may communicate the blood glucose trace directly to personal computing device 150 via wireless communication link 144. In alternate embodiments, the drug delivery device 105 may receive information regarding the blood glucose trace from sensor 180 via wireless communication link 146 and may relay that information to personal computing device 150 through wireless communication link 140.

The blood glucose trace may be analyzed to determine if the macronutrient profile of the meal was correct. If the blood glucose trace after a meal is suggestive that the meal nutrient or the portion sizes of the food items was not estimated correctly, feedback will be provided to the user via a user review tool 210 to help improve the estimation. For instance, if hyperglycemia is noted, it is likely that the total carbohydrate in the meal was mis-estimated (this could be a combination of a portion/nutrition error). On the other hand, if hypoglycemia is noted after the meal, it is suggestive of over estimation of the total carbohydrates (again a combination of a portion/nutrition error). If the nutrition information is known to be correct, the cause of the mis-estimation is likely a portion estimation error. Meals for which the blood glucose control have been good or bad may be stored in history information 251 and may be used by suggestion filter 202 to promote certain food items or combinations of food items or to indicate that certain food items or combinations of food items should be avoided for future meals.

The block diagram of the automatic insulin delivery system 100 shown in **FIG. 1** includes a block diagram of an exemplary drug delivery device 105 in accordance with the present invention as shown in **FIG. 1****.** In exemplary embodiments, drug delivery device 105 is configured to deliver bolus doses of insulin to a user over a predetermined period of time, for example, 72 hours. Drug delivery device 105 may also be capable of delivering basal doses of insulin. The drug delivery device 105 may implement (and/or provide functionality for) a drug delivery algorithm 106 to govern or control the automated delivery of bolus doses of insulin based on information received from macronutrient management application 156 running on personal computing device 150.

The drug delivery device 105 may be configured with a processor 102 which executes software stored in memory 104, such as drug delivery algorithm 106. The drug delivery algorithm 106 may be an application operable to cause the drug delivery device 105 to deliver bolus doses of insulin in accordance with pre-programmed parameters, for example, the overall quantity of insulation, the bolus split and the timing of the delivery of the bolus split.

Processor 102 may control a reservoir and pump 108 which is configured to pump the insulin from a reservoir to insulin delivery interface 110. In some embodiments, the reservoir and pump may be integrated into a single unit, while in other embodiments, the reservoir and pump may be separate units, wherein the pump is configured to draw the liquid drug from reservoir 108 and deliver it to the user via insulin delivery interface 110. In such an embodiment, the pump may be a semi-durable device, along with the processor, power source, communication interface, and memory, and this semi-durable device may be used with other reservoirs 108, and may comprise its own housing and be attachable to a housing of the reservoir and other disposable components of drug delivery device 105.

Insulin delivery interface 110 may comprise a needle or cannula for delivering the insulin into the body of the user (which may be done subcutaneously, intraperitoneally, or intravenously). Processor 102 may control insulin delivery interface 110 such as to cause the insulin delivery interface 110 to be inserted into the body of the user after the drug delivery device 105 has been attached to the body of the user. Programmable code for controlling the insertion of the insulin delivery interface 110 may be stored in memory 104 and executed by processor 102 and may be part of or separate from drug delivery algorithm 106.

In some embodiments, the drug delivery device 105 may include a communication interface 114 which may be a wireless transceiver that operates according to one or more radiofrequency protocols, such as Bluetooth, Wi-Fi, a near-field communication standard, a cellular standard, or the like.

In some embodiments, the drug delivery device 105 may optionally communicate, via communication interface 114, with personal computing device 150. Personal computing device 150 may be configured with a processor 152 and a memory 154 containing the macronutrient management application 156. The macronutrient management application 156 may be configured to provide information regarding the macronutrient profile of meals to drug delivery algorithm 106 via wireless communication link 140 such that drug delivery algorithm 106 may determine the proper bolus dose and bolus split for insulin delivery to the user over a predetermined period of time following ingestion of the meal by the user.

The drug delivery device 105, including all components previously discussed, are powered by power source 112, which may be, for example, one or more batteries or a power harvesting apparatus.

Automatic insulin delivery system 100 may further optionally include sensor 180 which may be, for example, a wearable continuous glucose monitor (CGM) which will provide blood glucose traces from the user directly to drug delivery device 105 via wireless communication link 146 or directly to personal computing device 150 via wireless communication link 144 for use by the macronutrient management application 156. Alternatively, macronutrient management application 156 may obtain the blood glucose traces from the drug delivery device 105 via wireless communication link 140 or blood glucose readings may be entered manually by the user to macronutrient management application 156.

Some examples of the disclosed system or methods may be implemented, for example, using a storage medium, a computer-readable medium, or an article of manufacture which may store an instruction or a set of instructions that, if executed by a machine (i.e., processor or controller), may cause the machine to perform a method and/or operation in accordance with examples of the disclosure. Such a machine may include, for example, any suitable processing platform, computing platform, computing device, processing device, computing system, processing system, computer, processor, or the like, and may be implemented using any suitable combination of hardware and/or software. The computer-readable medium or article may include, for example, any suitable type of memory unit, memory, memory article, memory medium, storage device, storage article, storage medium and/or storage unit, for example, memory (including non-transitory memory), removable or non-removable media, erasable or non-erasable media, writeable or re-writeable media, or the like. The instructions may include any suitable type of code, such as source code, compiled code, interpreted code, executable code, static code, dynamic code, encrypted code, programming code, and the like, implemented using any suitable high-level, low-level, object-oriented, visual, compiled and/or interpreted programming language. The non-transitory computer readable medium embodied programming code may cause a processor when executing the programming code to perform functions, such as those described herein.

Certain examples of the present disclosure were described above. It is, however, expressly noted that the present disclosure is not limited to those examples, but rather the intention is that additions and modifications to what was expressly described herein are also included within the scope of the disclosed examples. Moreover, it is to be understood that the features of the various examples described herein were not mutually exclusive and may exist in various combinations and permutations, even if such combinations or permutations were not made express herein, without departing from the spirit and scope of the disclosed examples. In fact, variations, modifications, and other implementations of what was described herein will occur to those of ordinary skill in the art without departing from the spirit and the scope of the disclosed examples. As such, the disclosed examples are not to be defined only by the preceding illustrative description.

The foregoing description of examples has been presented for the purposes of illustration and description. It is not intended to be exhaustive or to limit the present disclosure to the precise forms disclosed. Many modifications and variations are possible in light of this disclosure. It is intended that the scope of the present disclosure be limited not by this detailed description, but rather by the claims appended hereto. Future filed applications claiming priority to this application may claim the disclosed subject matter in a different manner and may generally include any set of one or more limitations as variously disclosed or otherwise demonstrated herein.

Although the present invention is defined in the attached claims, it should be understood that the present invention can also (alternatively) be defined in accordance with the following embodiments:
1. A method for providing information to a drug delivery device to enable post-prandial bolus dosing of insulin to a user, comprising:
   receiving meal information comprising one or more food items in a meal;
   determining a macronutrient profile for the meal; and
   using the macronutrient information to enable the drug delivery device to deliver a bolus dose as a bolus split of insulin to a user.
2. The method of embodiment 1 further comprising:
   providing the macronutrient profile of the meal to the drug delivery device;
   wherein the drug delivery device determines the bolus dose and bolus split based on the macronutrient profile of the meal.
3. The method of any of embodiments 1-2, further comprising:
   determining the bolus dose and bolus split based on the macronutrient profile; and providing the bolus dose and the bolus split information to the drug delivery device.
4. The method of any of embodiments 1-3, wherein the meal information comprises one or more food items selected as part of the meal further comprising:
   receiving user selections of the one or more food items via a meal selection interface;
   receiving information regarding a portion size of each food item via the meal selection interface; and
   storing the meal information in a meal catalog.
5. The method of any of embodiments 1-4, wherein the meal selection interface:
   provides an interface presenting graphical representations of suggested food items; and
   accepts selections of food items via a user selection of the graphical representations of the food items.
6. The method of any of embodiments 1-5 wherein receiving information regarding a portion size of each food item comprises:
   displaying graphical representations of small, medium and large portions of the food item; and
   receiving a user selection of one of the graphical representations of a portion of the food item.
7. The method of any of embodiments 1-6, wherein selections of food items presented in the meal selection interface are provided by a suggestion filter comprising:
   a machine leaning model trained to suggest one or more food items in the meal selection interface based on meal history information.
8. The method of any of embodiments 1-7, wherein the meal history information is selected from a group comprising past meals, locations associated with past meals and time-of-day associated with past meals.
9. The method of any of embodiments 1-8, wherein food item suggestions of the machine learning model are further based on location and time-of-day.
10. The method of any of embodiments 1-9, further comprising:
   receiving information regarding food items complementary to food items selected by the user; and
   including the complementary food items as food items suggested by the machine learning model.
11. The method of any of embodiments 1-10, wherein the determination of complementary food items is based on food items selected together by a large population of users.
12. The method of any of embodiments 1-11, wherein food item suggestions of the machine learning model are tailored based on one or more user preferences.
13. The method of any of embodiments 1-12, wherein the macronutrient profile of the meal comprises a quantity of the carbohydrate, fat and protein constituents of each of the one or more selected food items.
14. The method of any of embodiments 1-13, further comprising:
   receiving a post-prandial blood glucose trace of a user for a predetermined period of time after ingestion of the meal;
   determining accuracy of the macronutrient profile of the meal based on the blood glucose trace; and
   providing the accuracy information to the suggestion filter such that certain food items or combinations of food items may be promoted or avoided in future suggestions.
15. An automatic insulin delivery system comprising:
   a personal computing device
   a macronutrient management application executing on the personal computing device; and
   a drug delivery device, in wireless communication with the personal computing device;
   wherein the macronutrient management application:
      receives meal information comprising food items in a meal;
      determines a macronutrient profile for the meal; and
      provides the macronutrient profile to the drug delivery device to enable the drug delivery device to calculate and deliver a bolus dose as a bolus split of insulin to a user.
16. The system of embodiment 15 wherein the macronutrient management application comprises:
   a meal selection component for:
   providing an interface presenting graphical representations of suggested food items;
   receiving user selections of one or more of the food items; and
   receiving information regarding a portion size of each selected food item.
17. The system of any of embodiments 15-16, wherein the macronutrient management application further comprises:
   a suggestion filter component for providing food item suggestions to the meal selection interface component;
   wherein the suggestion filter component uses a machine learning model trained to provide the food item suggestions based on meal history information.
18. The system of any of embodiments 15-17, wherein food item suggestions of the machine learning model are further based on location and time-of-day.
19. The system of any of embodiments 15-18, wherein the macronutrient management application further comprises:
   a complementary association filter component for providing suggestions of food items complementary to user-selected food items to the meal selection interface;
   wherein determination of complementary food items is based on food items selected together by a large population of users.
20. The system of any of embodiments 15-19, wherein the macronutrient management application further comprises:
   a personalization filter component for providing user preferences to the suggestion filter, the user preferences comprising diet style preferences, cuisine preferences, individual food item preferences and allergy information; and
   a meal catalog component for storing and analyzing macronutrient profiles of previous meals.

## Claims

1. A method for providing information to a drug delivery device to enable post-prandial bolus dosing of insulin to a user, comprising:
receiving meal information comprising one or more food items in a meal;
determining a macronutrient profile for the meal; and
using the macronutrient information to enable the drug delivery device to deliver a bolus dose as a bolus split of insulin to a user.

2. The method of claim 1, further comprising:
providing the macronutrient profile of the meal to the drug delivery device;
wherein the drug delivery device determines the bolus dose and bolus split based on the macronutrient profile of the meal.

3. The method of any of claims 1-2, further comprising:
determining the bolus dose and bolus split based on the macronutrient profile; and
providing the bolus dose and the bolus split information to the drug delivery device.

4. The method of any of claims 1-3, wherein the meal information comprises one or more food items selected as part of the meal further comprising:
receiving user selections of the one or more food items via a meal selection interface, wherein the macronutrient profile preferably comprises a quantity of the carbohydrate, fat, and protein constituents of each of the one or more selected food items;
receiving information regarding a portion size of each food item via the meal selection interface; and
storing the meal information in a meal catalog.

5. The method of any of claims 1-4, wherein the meal selection interface:
provides an interface presenting graphical representations of suggested food items; and
accepts selections of food items via a user selection of the graphical representations of the food items.

6. The method of any of claims 1-5, wherein receiving information regarding a portion size of each food item comprises:
displaying graphical representations of small, medium and large portions of the food item; and
receiving a user selection of one of the graphical representations of a portion of the food item.

7. The method of any of claims 1-6, wherein selections of food items presented in the meal selection interface are provided by a suggestion filter comprising:
a machine leaning model trained to suggest one or more food items in the meal selection interface based on meal history information,
wherein the meal history information is optionally selected from a group comprising past meals, locations associated with past meals and time-of-day associated with past meals, and
wherein food item suggestions of the machine learning model are optionally further based on location and time-of-day or are optionally tailored based on one or more user preferences.

8. The method of any of claims 1-7, further comprising:
receiving information regarding food items complementary to food items selected by the user, wherein the determination of complimentary food items is preferably based on food items selected together by a large population of users; and
including the complementary food items as food items suggested by the machine learning model.

9. The method of any of claims 1-8, further comprising:
receiving a post-prandial blood glucose trace of a user for a predetermined period of time after ingestion of the meal;
determining accuracy of the macronutrient profile of the meal based on the blood glucose trace; and
providing the accuracy information to the suggestion filter such that certain food items or combinations of food items may be promoted or avoided in future suggestions.

10. An automatic insulin delivery system comprising:
a personal computing device
a macronutrient management application executing on the personal computing device; and
a drug delivery device, in wireless communication with the personal computing device;
wherein the macronutrient management application:
receives meal information comprising food items in a meal;
determines a macronutrient profile for the meal; and
provides the macronutrient profile to the drug delivery device to enable the drug delivery device to calculate and deliver a bolus dose as a bolus split of insulin to a user.

11. The system of claim 10, wherein the macronutrient management application comprises:
a meal selection component for:
providing an interface presenting graphical representations of suggested food items;
receiving user selections of one or more of the food items; and
receiving information regarding a portion size of each selected food item.

12. The system of any of claims 10-11, wherein the macronutrient management application further comprises:
a suggestion filter component for providing food item suggestions to the meal selection interface component;
wherein the suggestion filter component uses a machine learning model trained to provide the food item suggestions based on meal history information.

13. The system of any of claims 10-12, wherein food item suggestions of the machine learning model are further based on location and time-of-day.

14. The system of any of claims 10-13, wherein the macronutrient management application further comprises:
a complementary association filter component for providing suggestions of food items complementary to user-selected food items to the meal selection interface;
wherein determination of complementary food items is based on food items selected together by a large population of users.

15. The system of any of claims 10-14, wherein the macronutrient management application further comprises:
a personalization filter component for providing user preferences to the suggestion filter, the user preferences comprising diet style preferences, cuisine preferences, individual food item preferences and allergy information; and
a meal catalog component for storing and analyzing macronutrient profiles of previous meals.
